# EUROPÄISCHE PATENTSCHRIFT

(11) **EP 0 685 721 B1**
(45) Veröffentlichungstag und Bekanntmachung des Hinweises auf die Patenterteilung: **05.04.2000**
(21) Anmeldenummer: 95108407.8
(22) Anmeldetag: 01.06.1995
(51) Int. Cl.: G01L 19/00, G01L 9/00, A61M 1/14, G01L 7/08

(54) **Vorrichtung zum Messen des Drucks eines Mediums**
Device for measuring the pressure of a medium
Dispositif de mesure de la pression d'un médium

(30) Priorität: 03.06.1994 DE 4419593
(43) Veröffentlichungstag der Anmeldung: 06.12.1995
(73) Patentinhaber: Fresenius Medical Care Deutschland GmbH, 61350 Bad Homburg v.d.H. (DE)
(72) Erfinder: Wamsiedler, Ralf, 61440 Oberursel (DE); Wojke, Ralf Dr., 60431 Frankfurt (DE); Pieper, Walter, 61197 Florstadt1 (DE); Christmann-Braun, Horst, 65779 Kelkheim (DE)
(74) Vertreter: Vièl, Christof, Dipl.-Ing.

(56) Entgegenhaltungen:
- WO-A-86/02446
- FR-A- 2 125 991
- FR-A- 2 371 680
- US-A- 3 744 317

## Beschreibung

Die Erfindung betrifft eine Vorrichtung zum Messen des Drucks eines Mediums, bestehend aus einem Drucksensor mit einem ersten Druckübertragungsteil und aus einem zweiten Druckübertragungsteil, das mit dem Medium, dessen Druck zu messen ist, in Verbindung steht.

Derartige Vorrichtungen werden zum Messen von Drücken in Medien verschiedenster Art verwendet, z.B. in der Medizintechnik für die Druckbestimmung in extrakorporalen Blutkreisläufen, wie sie in der Dialysetechnik vorliegen.

Bei Vorrichtungen zum Messen des Drucks eines Mediums in einem Kreislauf, z.B. von Blut in extrakorporalen Blutkreisläufen, ist es nachteilig, wenn die Vorrichtung eine Abzweigung vom eigentlichen Kreislauf des Mediums für die Messung erfordert, da damit die einfache und übersichtliche Linienführung des Kreislaufs verlorengeht und mit der Komplexität die Fehlermöglichkeiten und die Aufbauzeit des Systems ansteigen. Zudem ist bei Vorliegen einer Abzweigung ein größeres Füllvolumen des Systems erforderlich. Aus Gründen der passiven Sicherheit ist jedoch ein möglichst geringes extrakorporales Blutvolumen wünschenswert. Schließlich entzieht ein Druckmessverfahren, das in einer Abzweigung des Blutkreislaufs arbeitet, diesem ein gewisses Blutvolumen und hält es relativ lange im Totraum zurück. Durch diese erhöhte Verweilzeit steigt die Gerinnungsneigung des Blutes und eine höhere Verabreichung von systemischen Koagulationshemmern, wie Heparin, wird notwendig.

Diese Nachteile entfallen bei nichtinvasiven Systemen. Erfolgt die Druckmessung statt an einer Ableitung direkt am Schlauch, so bleibt die Linienführung des Schlauchsystems übersichtlich, das Füllvolumen steigt nur unwesentlich und der Totraum der Ableitung entfällt.

Schläuche, wie z.B. Blutschläuche, haben sowohl elastische als auch viskose bzw. visko-elastische Eigenschaften. Während nach einer elastischen Beanspruchung der Ausgangszustand wieder erreicht wird, ist dies bei einer viskosen Änderung nicht der Fall. Eine absolute Druckmessung am Schlauch wird insbesondere durch diese viskosen Eigenschaften des Schlauchmaterials erschwert. Eine Möglichkeit, den Einfluß der Viskosität des Materials zu minimieren, ist die Verwendung einer dünnen Membran statt eines dicken Schlauchs im Bereich der Druckmeßstelle. Es ergibt sich jedoch dabei das Problem der Übertragung der Membranbewegung auf den Drucksensor. Eine weitere Schwierigkeit liegt in der Tatsache, daß in der Regel auch sehr hohe negative Drücke erfaßt werden sollen.

Aus der EP 0 355 373 B1 ist ein Fluid-Druckwandler bekannt, bei dem eine Meßvorrichtung die Ausdehnung oder Schrumpfung eines kissenartigen Körpers, der sich je nach dem Druck des in dem kissenartigen Körper fließenden Fluids ausdehnen oder zusammenziehen kann, erfaßt. Die Messung an der gewölbten Oberfläche des kissenartigen Körpers erfolgt mit Hilfe einer daran befestigten Eisenplatte über einen Magneten, der an der Spitze der Meßvorrichtung lösbar befestigt ist. Diese Vorrichtung ist aufwendig, da die Eisenplatte mit dem von dem Medium durchströmten Teil verbunden ist und daher bei Einwegsystemen, wie z.B. einem extrakorporalen Blutkreislauf, nach einmaliger Benutzung zu kostspielig wäre. Zudem ist die Sensibilität eines derartigen Systems durch die ihm inhärente Trägheit relativ gering.

In der EP 0 330 891 B1 wird eine Einrichtung zur Druckübertragung beschrieben, bei der ein Außengehäuse durch eine Membran in zwei getrennte Räume geteilt ist, von denen der eine von dem Medium, dessen Druck bestimmt werden soll, und der andere von einem Hilfsmedium durchströmt wird, wobei die Membran die Druckschwankungen des Mediums an das Hilfsmedium weitergibt, an dem die eigentliche Druckmessung in bekannter invasiver Weise erfolgt. Auch hier liegt aufgrund der indirekten Vorgehensweise und trägheitsbedingt ein nicht sehr sensibles und relativ langsam reagierendes System vor.

Auch bei dem Druckmeßsystem, wie es in der WO 93/22641 A1 beschrieben ist, werden die Druckänderungen von der Membran auf den Drucksensor mittels eines Fluids übertragen, wobei die oben beschriebenen Nachteile auftreten. Zudem ist dieses System wie auch das vorher beschriebene relativ komplex und fertigungstechnisch aufwendig, weshalb beide Vorrichtungen insbesondere für die Verwendung in Einwegsystemen nicht in Frage kommen.

Ebenso wird in der WO 86/02446 ein Druckmeßsystem beschrieben, bei dem Druckänderungen über eine Membran und ein Fluid auf ein Druckmeßelement übertragen werden. Auch dieses System dürfte aufgrund seines komplexen Aufbaus kaum für Einwegsysteme, mit denen schnelle und präzise Messungen erfolgen sollen, in Frage kommen.

Aus der EP 0 200 709 B1 ist ein Druckaufnehmer mit einer mit einem Gehäuse fest verbundenen und dieses gegen das zu vermessende Medium abdichtenden Plattenmembran, welche mit dem Meßelement zur Übertragung des zu messenden Drucks in Verbindung steht, bekannt. Ein ringförmiger Bereich der Membran ist dabei im Urzustand gewölbt oder konisch ausgebildet. Diese Ausbildung der Membran bringt einen erhöhten Fertigungsaufwand mit sich und verschlechtert den Kontakt zwischen Sensor- und Schlauchmembran.

Die EP 0 130 441 B1 zeigt ein Druckübertragungssystem, welches ohne Übertragungsflüssigkeit auskommt, Hierbei wird der Hohlraum zwischen den beiden Druckübermittlungsteilen mit einer Vakuumpumpe leergesaugt, wodurch die Druckübermittlungsteile in Kontakt gebracht werden. Dieses System ist überaus anfällig, sowie sehr aufwendig und somit teuer. Darüber hinaus wird das System über einen Schlauch angekoppelt, so daß das Fluid durch die Meßvorrichtung fließen muß, was die oben beschriebenen Nachteile eines invasiven Systems mit sich bringt.

Aus der DE-C-29 30 869 ist eine Druckmeßkapsel bekannt, bei der zwei Membranen durch Verdrehen von Gehäuseteilen in Kontakt gebracht werden. Da davon auszugehen ist, daß die Druckmeßkapsel und der Meßwandler manuell verschraubt werden, sind die erreichbaren Anpreßkräfte zwischen den beiden Membranen relativ gering. Zudem führt das Aufbringen durch Drehbewegung zu einer Membrantorsion, die der geforderten Dichtigkeit abträglich ist und sogar zur Zerstörung der Membran führen kann, was wiederum im medizinischen Anwendungsfeld unter allen Umständen vermieden werden muß.

Schließlich ist aus der US-A-3,744,317 eine Druckmeßvorrichtung bekannt, bei der ein in einem ersten Körper angeordneter O-Ring gegen eine in einem zweiten Körper angeordnete Membran gedrückt wird, welche wiederum gegen eine Meßmembran oder -wand gedrückt wird, die Dehnungsmeßstreifen aufweist. Das Verspannen der beiden Körper erfolgt mittels Verbindungsschrauben. Diese Vorrichtung ist insofern von Nachteil, als negative Drücke nicht gemessen werden können, da bei negativen Drücken eine Ablösung der Membran von der Meßmembran erfolgt.

Aufgabe der Erfindung ist es, eine gattungsgemäße Vorrichtung zum Messen des Drucks eines Mediums zu schaffen, mit der auf einfache Weise im Rahmen einer nichtinvasiven Messung sowohl positive als auch negative Drücke möglichst präzis und schnell erfaßt werden können. Das System soll leicht herstell- und handhabbar sein und auch für Einwegsysteme geeignet sein.

Diese Aufgabe wird erfindungsgemäß durch eine Vorrichtung gemäß Anspruch 1 gelöst. Vorteilhafte Ausbildungen der Vorrichtung sind in den abhängigen Ansprüchen 2 bis 11 definert. Eine Verwendung der Vorrichtung gemäß Anspruch 1 entnimmt man den Ansprüchen 12 bis 14. Mit einer derartigen Vorrichtung kann aufeinfache Weise ein Kontakt zwischen den beiden Druckübertragungsteilen hergestellt werden, der eine trägheitsarme, nichtinvasive Messung positiver und negativer Drücke ermöglicht.

Durch die translatorische Krafteinwirkung in axialer Richtung sind hohe Anpreßdrücke und somit eine gute Randdichtigkeit erreichbar, wie sie für die Unterdruckmessung erforderlich ist. Zudem entsteht durch die Krafteinwirkung in axialer Richtung keine Membrantorsion, die der für den Unterdruckbereich geforderten Dichtigkeit des Raumes zwischen den Membranen abträglich ist und auch zur Zerstörung der Membranen führen kann.

Eine Weiterbildung der Erfindung besteht darin, daß das erste Druckübertragungsteil in seinem Randbereich derart gegen das zweite Druckübertragungsteil preßbar ist, daß der Raum zwischen den beiden Druckübertragungsteilen nach außen hin abgedichtet ist und die beiden Druckübertragungsteile ohne Luft- oder Flüssigkeitseinschluß direkt übereinanderliegen.

Es liegt zwischen den Druckübertragungsteilen ein Vakuum vor, das eine praktisch trägheitsfreie Druckübertragung ermöglicht.

Dadurch müssen in einem Einwegsystem lediglich das zweite Druckübertragungsteil, also die Membran, sowie die Spannvorrichtung ausgewechselt werden, wenn die Einwegbestandteile des Systems gewechselt werden. Diese Teile können kostengünstig hergestellt und auch einem Recycling zugeführt werden.

Es ist sinnvoll, daß eine Führung zur Aufnahme und zum Anpressen des ersten Druckübertragungsteils vorgesehen ist.

Durch das Einführen des ersten Druckübertragungsteils, das mit dem Sensor verbunden ist, wird das System betriebsbereit gemacht.

Eine Variante der erfindungsgemäßen Vorrichtung sieht vor, daß das zweite Druckübertragungsteil in der Spannvorrichtung über der Öffnung der Spannvorrichtung gehalten wird.

Weiterhin ist es möglich, daß das zweite Druckübertragungsteil über der Öffnung der Spannvorrichtung mittels einer um die Öffnung verlaufenden Erhebung gespannt wird.

Dabei kann die Erhebung durch einen O-Ring gebildet werden.

Es ist sinnvoll, daß das zweite Druckübertragungsteil, die Spannvorrichtung und gegebenenfalls die Erhebung an einer Druckmeßkammer mit zwei Schlauchanschlüssen angeordnet sind.

Dabei ist es zweckmäßig, daß die Druckmeßkammer eine zylindrische Form aufweist.

Weiterhin ist es sinnvoll, daß in dem Bereich der Druckmeßkammer, der dem zweiten Druckübertragungsteil gegenüberliegt, eine volumenreduzierende Erhebung vorliegt.

Vorteilhaft ist, daß die volumenreduzierende Erhebung kegelstumpfförmig ausgebildet ist.

Erfindungsgemäß ist die Verwendung der Vorrichtung allgemein zur nichtinvasiven Druckmessung, z.B. an einem Blutschlauchsystem, insbesondere dem Blutschlauchsystem einer Dialysemaschine.

Im folgenden wird ein Beispiel einer erfindungsgemäßen Vorrichtung anhand von Zeichnungen beschrieben. Es zeigen
- Fig. 1: eine geschnittene schematische Darstellung einer erfindungsgemäßen Vorrichtung,
- Fig. 2, Fig. 3: jeweils eine Seitenansicht und Draufsicht von Druckmeßkammern.
- Fig. 4: eine geschnittene Darstellung eines Ausführungsbeispiels einer erfindungsgemäßen Vorrichtung,
- Fig. 5: eine Darstellung der Ausgangsspannung in Abhängigkeit von dem Druck im Medium, ermittelt mit der erfindungsgemäßen Vorrichtung.

In der Regel wird die erfindungsgemäße Vorrichtung an einer Druckmeßkammer 8 angeordnet sein, die zwei Anschlüsse 9 für den Schlauch aufweist, in dem das zu messende Medium (in Pfeilrichtung) zirkuliert.

Die Vorrichtung besteht aus einem zweiten Druckübertragungsteil 2, das in der Regel eine Membran darstellt, die auch als Schlauchmembran bezeichnet wird. Verschiedenste Materialien können für die Herstellung dieser Membran verwendet werden, soweit sie mit dem zu fördernden Medium verträglich, d.h. im beschriebenen Beispiel blutverträglich sind, die Elastizität und Druckfestigkeit im jeweiligen Druckbereich ausreichend sind; sich keine bleibende Verformung einstellt und die erforderliche Dichtigkeit gegeben ist. Dies ist beispielsweise bei polyurethanbeschichtetem Latex, SI, PU, Polyolefinen, PETB und PVC der Fall. In der Regel wird das zweite Druckübertragungsteil 2 eine runde Form aufweisen, jedoch sind auch andere Geometrien denkbar.

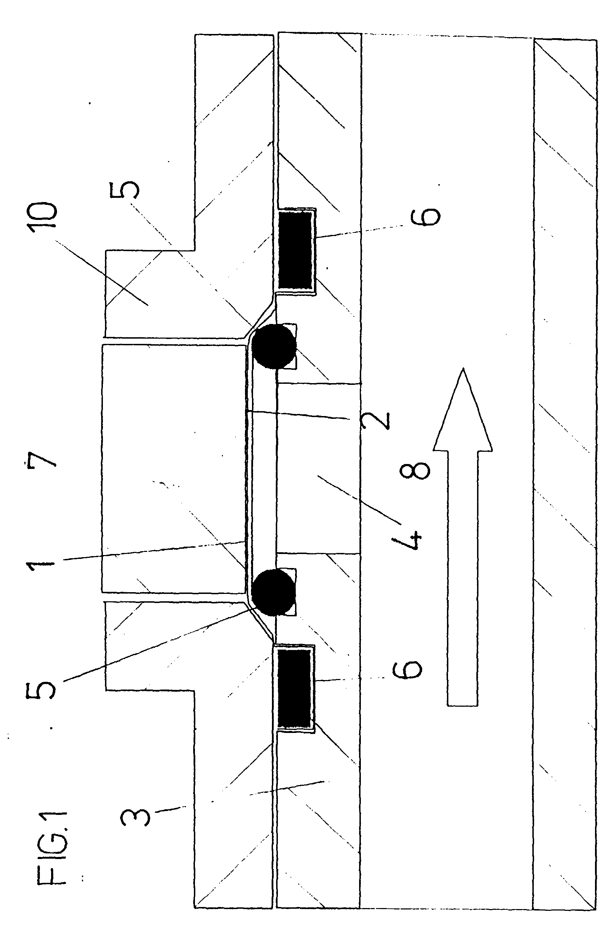

Das zweite Druckübertragungsteil 2 wird über eine Öffnung 4 in einer Spannvorrichtung 3 glatt aufgespannt, was z.B. mittels eines Spannrings 6, wie in Fig. 1 dargestellt, erfolgen kann. Dabei können das zweite Druckübertragungsteil 2 und der Spannring 6 auch zwischen dem Medium und der Oberseite der Spannvorrichtung 3 abdichtend wirken. Es besteht allerdings auch die Möglichkeit, das zweite Druckübertragungsteil 2 auf der Spannvorrichtung 3 aufzukleben, wenn die jeweiligen Materialien dies erlauben.

Das zweite Druckübertragungsteil 2 kann glatt auf der Oberkante der Spannvorrichtung 3 und über der Öffnung 4 liegen oder, wie in Fig. 1 dargestellt, über eine um die Öffnung 4 verlaufende Erhebung 5 gespannt werden. Diese Erhebung 5 kann auf einfache Weise durch einen in einer Ringnut liegenden O-Ring gebildet werden. Durch die Elastizität der Erhebung 5 und der dünnen Schlauchmembran 2 wird eine plane Oberfläche im angehobenen Bereich des zweiten Druckübertragungsteils 2 erreicht. Liegt keine Erhebung vor, so kann es sinnvoll sein, die obere Kante der Öffnung 4 in der Spannvorrichtung 3 abzufasen, um eine Verletzung des zweiten Druckübertragungsteils 2 bei dessen Bewegung zu vermeiden.

Auf den über der Öffnung 4 und somit von den Druckschwankungen des Mediums beeinflußten Bereich der Schlauchmembran 2 und eventuell einen darüber hinausgehenden, über der Spannvorrichtung 3 liegenden Bereich wird nun ein erstes Druckübertragungsteil 1, das in der Regel ebenfalls eine Membran darstellt, zumindest im Randbereich des zweiten Druckübertragungsteils 2 derart gepreßt, daß die beiden Druckübertragungsteile 1, 2 dicht aufeinanderliegen und der zwischen den Druckübertragungsteilen 1, 2 eingeschlossene Raum möglichst klein ist, d.h. gegen Null geht. Idealerweise ist der Luftspalt zwischen den beiden Druckübertragungsteilen 1, 2 gleich Null. Da der Bereich zwischen den beiden Druckübertragungsteilen 1, 2 keine Verbindung zur Atmosphäre hat und eventuell dort ein Vakuum vorliegt, haften die beiden Druckübertragungsteile 1, 2 aufeinander, das flexible Zentrum der Druckübertragungsteile 1, 2 wird abgedichtet und diese bewegen sich synchron in Abhängigkeit vom Druck des Mediums, so daß sowohl positive als auch negative Drücke von dem zweiten Druckübertragungsteil 2 auf das erste Druckübertragungsteil 1 übertragen werden können. Da die Übertragung der Druckschwankungen zwischen den beiden Druckübertragungsteilen 1, 2 ohne Zwischenschaltung einer Flüssigkeit erfolgt, ist die Trägheit der erfindungsgemäßen Vorrichtung gering und die Sensibilität und Genauigkeit der Messung sind größer.

Es ist dafür erforderlich, daß die beiden Druckübertragungsteile 1, 2 in ihrer Geometrie aufeinander angepaßt sind. Das erste Druckübertragungsteil 1 kann ebenfalls aus den verschiedensten Materialien bestehen, z.B. kann es eine Edelstahlmembran sein oder auch aus Keramik bestehen. Das erste Druckübertragungsteil 1 geht entweder direkt oder indirekt in den Drucksensor 7 über, der die Druckänderungen des Mediums aufnimmt. Dieser Drucksensor 7 läuft beispielsweise in einer Führung 10 und wird entweder im Bereich der Erhebung 5 oder des planen Randbereichs um die Öffnung 4 der Spannvorrichtung 3 über das erste Druckübertragungsteil 1 auf das zweite Druckübertragungsteil 2 gedrückt, wobei die Erhebung 5 oder der Randbereich um die Öffnung 4 als Gegenlager dienen. Insbesondere im Bereich des Gegenlagers muß die Anpressung ausreichend stark sein, um einen direkten Kontakt der Druckübertragungsteile 1, 2 zu gewährleisten.

Es ist vorteilhaft, wenn, wie in Fig. 1 dargestellt, die Führung 10 des Drucksensors 7 gleichzeitig zur Klemmung des Spannrings 6 dient.

Das zweite Druckübertragungsteil 2 sollte möglichst plan angeordnet sein. Beim Anbringen einer planen (Kreis-)Fläche seitlich an ein zylindrisches Rohr (den Schlauch mit dem zu messenden Medium) entstehen jedoch prinzipiell Übergänge oder Toträume. Diese können jedoch klein gehalten werden, wenn die Abmessungen des zweiten Druckübertragungsteils 2 nicht wesentlich größer als der Schlauchdurchmesser sind.

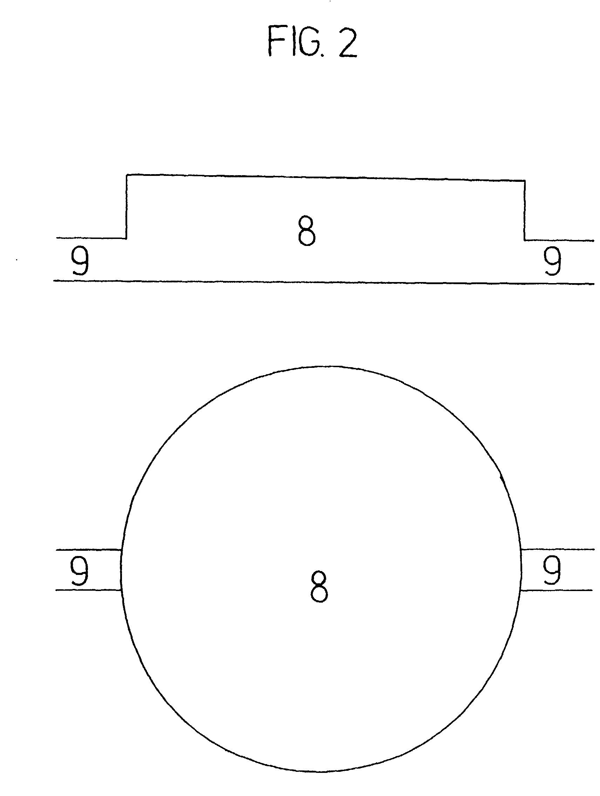

Fig. 2 zeigt eine einfache Form einer Druckmeßkammer 8 mit Schlauchanschlüssen 9, die eine zylindrische Form aufweist. Die Schlauchmembran 2 und die Sensormembran 1 werden vorzugsweise im oberen Bereich der Druckmeßkammer 8 aufeinandergepreßt. Diese Druckmeßkammer 8 weist ein relativ großes Volumen und einen deutlich größeren Querschnitt als der Schlauch auf, weshalb die Fließgeschwindigkeit verhältnismäßig gering sein wird. Bei Flüssigkeiten wie Blut, bei denen eine Verlängerung der Durchlaufzeit Veränderungen, hier Gerinnung, mit sich bringen kann, sind Toträume ungünstig.

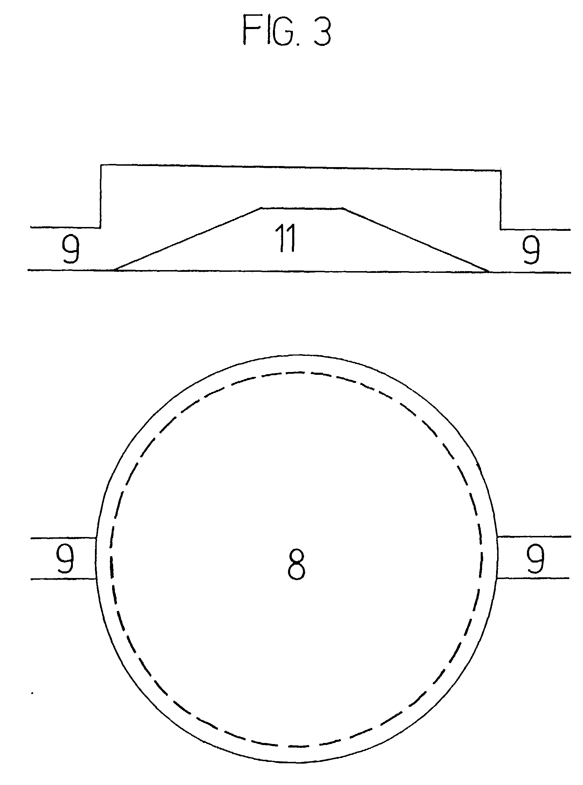

Fig. 3 zeigt eine Möglichkeit, das Volumen der Druckmeßkammer 8 zu vermindern. Hierzu wird in der Druckmeßkammer 8 eine volumenreduzierende Erhebung 11 vorgesehen, was zweckmäßigenveise an der der Membran 2 gegenüberliegenden Seite der Druckmeßkammer 8 erfolgt. Bei einer zylindrischen Druckmeßkammer 8, wie sie hier dargestellt ist, bietet sich als volumenreduzierende Erhebung 11 ein Kegel, bzw. wie in Fig. 3 gezeigt, ein Kegelstumpf oder auch eine Halbkugel an. Je nach Form der Druckmeßkammer 8 kann jedoch die Form der volumenreduzierenden Erhebung 11 variiert werden.

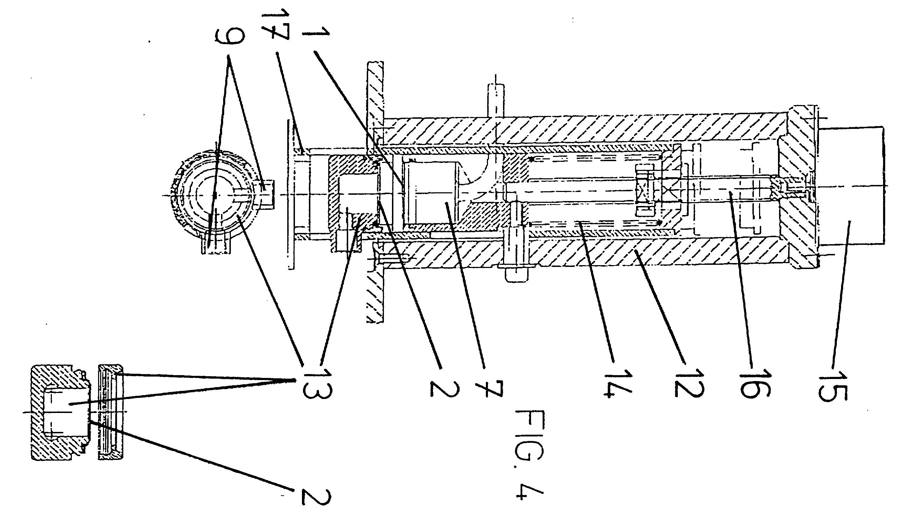

In Fig. 4 ist eine beispielhafte Ausführungsform einer erfindungsgemäßen Vorrichtung für die Druckbestimmung in extrakorporalen Kreisläufen dargestellt. Die Komponenten einer derartigen Vorrichtung können in zwei Gruppen unterteilt werden. Die erste befindet sich im extrakorporalen Kreislauf und besteht aus einem Gehäuse, das im vorliegenden Fall aus einem Deckel und dem Gehäusekörper mit zwei Schlauchanschlüssen 9, auf dem sich stirnseitig eine leicht vorgespannte Membran 2, z.B. eine Silikonmembran, befindet. Diese erste Gruppe wird auch als Disposable 13 bezeichnet.

Die zweite Einheit befindet sich auf der Geräteseite. Sie besteht aus einem Gehäuse 12, in dem ein Drucksensor 7 in einer Aufnahme angeordnet ist. Über diese Aufnahme kann der Drucksensor 7 mit Hilfe eines federnden Elements 14, z.B. einer Spiralfeder, über eine Membran 1 an die Membran 2 des Disposables 13 angekoppelt bzw. über einen Antrieb 15 (z.B. elektrisch oder pneumatisch) und, eventuell, wie hier gezeigt, über eine Spindel 16 wieder entkoppelt werden.

Bei der Ankopplung wird der Drucksensor 7 stirnseitig mit dem äußeren Bereich seiner Membran 1 auf den Rand der Membran 2, unter dem sich ein Widerlager befindet, gepreßt. Das Disposable 13 ist so ausgelegt, daß der Raum zwischen den Membranen 1 und 2 zur Umgebung hin abgedichtet ist und dieser Raum möglichst klein ist, idealerweise Null. Durch diese Maßnahme wird sichergestellt, daß selbst bei hohen Unterdrücken eine vollständige Dichtigkeit und somit eine hohe Meßgenauigkeit gegeben ist.

In der dargestellten Vorrichtung ist eine Schubladenvorrichtung 17 zur Aufnahme des Disposables 13 vorgesehen. Die Schubladenvorrichtung 17 kann sich in drei Stellungen befinden. Zur Aufnahme des Disposables 13 wird sie vollständig ausgefahren. Für den Betrieb fährt die Schubladenvorrichtung 17 mit dem Disposable 13 bis zum Drucksensor 7 zurück und koppelt die Membranen 1 und 2 aneinander. Ist die Druckmeßeinheit nicht in Betrieb, ist die Schubladenvorrichtung 17 komplett eingefahren. Dabei schließt sie frontbündig mit der Gerätefrontplatte, was für die Reinigung der Vorrichtung von Vorteil ist.

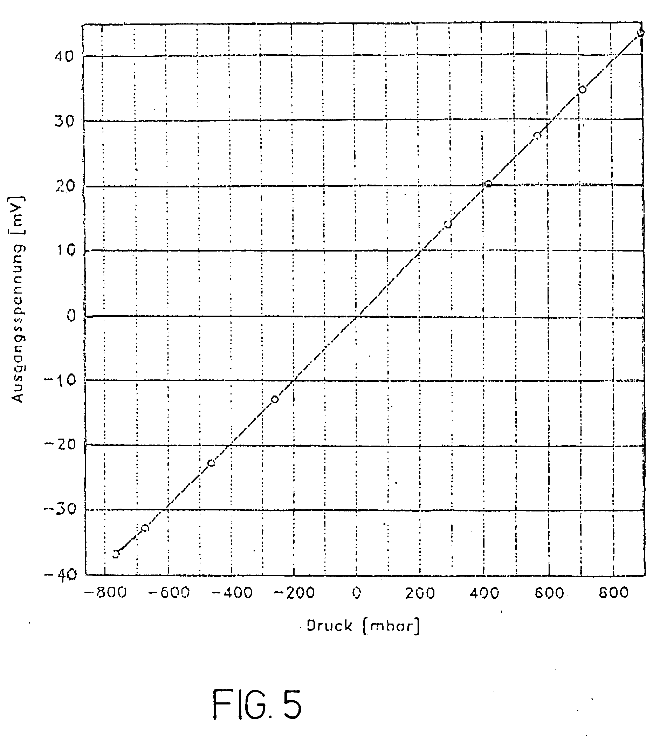

In Fig. 5 ist schließlich gezeigt, daß über einen weiten Bereich des Mediumdrucks die Ausgangsspannung des Drucksensors 7, trotz zwischengeschalteter Membran 2, linear verläuft. Die sehr präzisen Meßergebnisse, auch im negativen Druckbereich, sind der guten Haftung der beiden aneinandergekoppelten Membranen 1, 2 zu verdanken.

## Patentansprüche

1. Vorrichtung zum Messen des Drucks eines Mediums, bestehend aus einem Drucksensor (7) mit einem ersten Druckübertragungsteil (1), und aus einem zweiten Druckübertragungsteil (2), das mit dem Medium, dessen Druck zu messen ist, in Verbindung steht, wobei der die Druckänderungen des Mediums aufnehmende Drucksensor (7) durch translatorische Krafteinwirkung in axialer Richtung über das erste Druckübertragungsteil (1) an das sich mit diesem synchron in Abhängigkeit vom Druck bewegende zweite Druckübertragungsteil (2) anpreßbar ist, wobei der Randbereich um die Öffnung (4) als Gegenlager dient, wobei das zweite Druckübertragungsteil (2) mittels eines Spannrings (6) über einer Öffnung (4) einer Spannvorrichtung (3) gehalten wird, durch welche das zweite Druckübertragungsteil (2) mit dem Medium in Verbindung treten kann.

2. Vorrichtung gemäß Anspruch 1, wobei das erste Druckübertragungsteil (1) in seinem Randbereich derart gegen das zweite Druckübertragungsteil (2) preßbar ist, daß der Raum zwischen dem ersten und dem zweiten Druckübertragungsteil (1, 2) nach außen hin abgedichtet ist und die beiden Druckübertragungsteile (1, 2) ohne Luft- oder Flüssigkeitseinschluß direkt übereinanderliegen.

3. Vorrichtung gemäß einem der Ansprüche 1 und 2 wobei eine Führung (10) zur Aufnahme und zum Anpressen des ersten Druckübertragungsteils (1) vorgesehen ist.

4. Vorrichtung gemäß Anspruch 1, wobei das zweite Druckübertragungsteil (2) über der Öffnung (4) der Spannvorrichtung (3) mittels einer um die Öffnung (4) verlaufenden Erhebung (5) gespannt wird.

5. Vorrichtung gemäß Anspruch 4, wobei die Erhebung (5) durch einen O-Ring gebildet wird.

6. Vorrichtung gemäß einem der Ansprüche 1 bis 5 wobei das zweite Druckübertragungsteil (2), die Spannvorrichtung (3) und gegebenenfalls die Erhebung (5) an einer Druckmeßkammer (8) mit zwei Schlauchanschlüssen (9) angeordnet sind.

7. Vorrichtung gemäß Anspruch 6, wobei die Druckmeßkammer (8) eine zylindrische Form aufweist.

8. Vorrichtung gemäß Anspruch 7, wobei in dem Bereich der Druckmeßkammer (8), der dem zweiten Druckübertragungsteil (2) gegenüberliegt, eine volumenreduzierende Erhebung (11) vorliegt.

9. Vorrichtung gemäß Anspruch 8, wobei die volumenreduzierende Erhebung (11) kegelstumpfförmig ausgebildet ist.

10. Vorrichtung gemäß einem der Ansprüche 1 bis 9, wobei die Druckübertragungsteile (1, 2) Membranen sind.

11. Vorrichtung gemäß Anspruch 10, wobei das Druckübertragungsteil (2) eine Silikonmembran ist.

12. Verwendung einer Vorrichtung gemäß einem der Ansprüche 1 bis 16 zur nichtinvasiven Druckmessung.

13. Verwendung gemäß Anspruch 17 zur nichtinvasiven Druckmessung an einem Blutschlauchsystem.

14. Verwendung gemäß Anspruch 18 zur nichtinvasiven Druckmessung an dem Blutschlauchsystem einer Dialysemaschine.

## Revendications

1. Dispositif de mesure de la pression d'un milieu, se composant d'un capteur de pression (7) ayant un premier élément de transmission de la pression (1) et d'un deuxième élément de transmission de la pression (2) qui est en contact avec le milieu dont la pression est à mesurer, le capteur de pression (7) enregistrant les variations de pression du milieu pouvant être appuyé sous l'action d'une force de translation en direction axiale par le premier élément de transmission de la pression (1) contre le deuxième élément de transmission de la pression (2) se déplaçant avec celui-ci de façon synchrone en fonction de la pression, le bord périphérique autour de l'ouverture (4) servant de contre-portée, le deuxième élément de transmission de la pression (2) étant maintenu au moyen d'un anneau tendeur (6) sur une ouverture (4) d'un dispositif de fixation (3) au travers duquel le deuxième élément de transmission de la pression (2) peut entrer en contact avec le milieu.

2. Dispositif selon la revendication 1, le premier élément de transmission de la pression (1) pouvant être appuyé au niveau de son bord périphérique contre le deuxième élément de transmission de la pression (2) de sorte que l'espace entre le premier et le deuxième élément de transmission de la pression (1, 2) est rendu étanche vis-à-vis de l'extérieur, et les deux pièces de transmission de la pression (1, 2) étant posées l'une sur l'autre sans inclusion d'air ou de liquide.

3. Dispositif selon l'une des revendications 1 et 2, un guide (10) étant prévu pour recevoir et appuyer le premier élément de transmission de la pression (1).

4. Dispositif selon la revendication 1, le deuxième élément de transmission de la pression (2) pouvant être tendu au-dessus de l'ouverture (4) du dispositif de fixation (3) au moyen d'une saillie (5) s'étendant autour de l'ouverture (4).

5. Dispositif selon la revendication 4, la saillie (5) étant formée par un joint circulaire.

6. Dispositif selon l'une des revendications 1 à 5, le deuxième élément de transmission de la pression (2), le dispositif de fixation (3) et le cas échéant la saillie (5) étant disposés sur une chambre de mesure de pression (8) avec deux raccords de tuyau (9).

7. Dispositif selon la revendication 6, la chambre de mesure de pression (8) présentant une forme cylindrique.

8. Dispositif selon la revendication 7, une saillie réductrice de volume (11) étant prévue dans le domaine de la chambre de mesure de pression (8) située à l'opposée du deuxième élément de transmission de la pression (2).

9. Dispositif selon la revendication 8, la saillie réductrice de volume (11) étant tronconique.

10. Dispositif selon l'une des revendications 1 à 9, les pièces de transmission de la pression (1, 2) étant des membranes.

11. Dispositif selon la revendication 10, la pièce de transmission de la pression (2) étant une membrane en silicone.

12. Utilisation d'un dispositif selon l'une des revendications 1 à 11 pour la mesure non invasive de la pression.

13. Utilisation selon la revendication 12 pour la mesure non invasive de la pression sur un système de tuyaux de circulation sanguine.

14. Utilisation selon la revendication 13 pour la mesure non invasive de la pression sur un système de tuyaux de circulation sanguine d'une machine de dialyse.

## Claims

1. A device for measuring the pressure of a medium, consisting of a pressure sensor (7) with a first pressure transmission element (1), and of a second pressure transmission element (2) in contact with the medium whose pressure is to be measured, it being possible by means of the translatory action of force in axial direction for the pressure sensor (7), which picks up the pressure fluctuations in the medium, to press via the first pressure transmission element (1) against the second pressure transmission element (2), which moves synchronously with the first pressure transmission element (1) as a function of the pressure, with the edge zone surrounding the opening (4) serving as support and the second pressure transmission element (2) being held by means of a clamping ring (6) over an opening (4) in a clamping device (3), through which opening the second pressure transmission element (2) can make contact with the medium.

2. The device of claim 1, it being possible to press the edge zone of the first pressure transmission element (1) in such a way against the second pressure transmission element (2) that the space between the first and the second pressure transmission elements (1,2) is sealed off from the outside and the two pressure transmission elements (1,2) are directly superimposed on one another without the inclusion of air or liquid.

3. A device according to one of claims 1 and 2, a guide means (10) being provided for the accommodation and pressing on of the first pressure transmission element (1)

4. The device of claim 1, the second pressure transmission element (2) being clamped over the opening (4) in the clamping device (3) by means of an elevation (5) surrounding the opening (4).

5. The device of claim 4, the elevation (5) being formed by an O-seal.

6. A device according to one of claims 1 to 5, the second pressure transmission element (2), the clamping device (3) and maybe the elevation (5) being disposed on a pressure measuring chamber (8) provided with two tube connections (9).

7. The device of claim 6, the pressure measuring chamber (8) being circular in shape.

8. The device of claim 7, a volume-reducing elevation (11) being provided in the pressure measuring chamber (8), in the part which lies opposite the second pressure transmission element (2).

9. The device of claim 8, the volume-reducing elevation (11) having the shape of a truncated cone.

10. A device according to one of claims 1 to 9, the pressure transmission elements (1,2) being membranes.

11. The device of claim 10, the pressure transmission element (2) being a silicone membrane.

12. Use of a device according to one of claims 1 to 11 for non-invasive pressure measurement.

13. Use according to claim 12 for non-invasive pressure measurement in systems of tubing carrying blood.

14. Use according to claim 13 for non-invasive pressure measurement in systems of tubing for dialysis machines.
